# EUROPÄISCHE PATENTSCHRIFT

(11) **EP 1 658 871 B1**
(45) Veröffentlichungstag und Bekanntmachung des Hinweises auf die Patenterteilung: **13.02.2008**
(21) Anmeldenummer: 05024052.2
(22) Anmeldetag: 04.11.2005
(51) Int. Cl.: A61M 5/162

(54) **Vorrichtung zum Einleiten von Luft in bei der künstlichen Ernährung verwendete Behälter**
Device for venting containers used in enteral feeding
Dispositif de ventilation pour des conteneurs à usage dans l'alimentation entérale

(30) Priorität: 22.11.2004 DE 202004018089 U; 30.06.2005 DE 202005010459 U
(43) Veröffentlichungstag der Anmeldung: 24.05.2006
(73) Patentinhaber: Filtertek B.V., County of Limerick (IE)
(72) Erfinder: Hogan, Brendan, Gort (IE); Scheu, Rolf Rainer, 60488 Frankfurt am Main (DE)
(74) Vertreter: Brose, D. Karl

(56) Entgegenhaltungen:
- EP-A- 0 379 047
- EP-A- 1 088 765
- US-A- 4 793 503
- US-A- 5 125 522
- US-A1- 2004 153 047

## Beschreibung

Die Erfindung betrifft eine Vorrichtung zum Einleiten von Luft in bei der künstlichen Ernährung verwendete Behälter, mit einem Ventil, welches in einem, das Enteralbesteck mit dem die Enteralflüssigkeit enthaltenden Behälter verbindenden Anschlussteil angeordnet ist und die Luftzufuhr entsprechend dem Verbrauch der Enteralflüssigkeit regelt.

Da durch derartige Ventile die Luftzufuhr entsprechend dem Verbrauch beim Einleiten der Enteralflüssigkeit in den Patienten geregelt wird, ist gewährleistet, dass die Schwerkraftförderung der Enteralflüssigkeit ohne Schwierigkeiten abläuft. Bisher hat man in den üblicherweise durch auf den Behälter aufschraubbare Kappen gebildeten Anschlussteilen Rückschlagventile vorgesehen, welche jedoch nicht in allen Fällen zufriedenstellend arbeiten. Es kommt hinzu, dass Enteralflüssigkeiten üblicherweise in Behältern geliefert werden, welche zusätzlich zu der den Verschluss bildenden Schraubkappe durch eine aufgeschweißte Aluminium- oder Kunststofffolie verschlossen sind. In der Praxis muss daher eine derartige Verschlussfolie in einem getrennten Arbeitsschritt geöffnet werden, ehe das Enteralbesteck durch Aufschrauben oder Aufdrücken der Anschlussteile in Betrieb genommen werden kann.

Der Erfindung liegt die Aufgabe zugrunde, eine Vorrichtung der eingangs genannten Art derartig auszugestalten, dass einerseits eine absolut funktionssichere Arbeitsweise des Ventils gewährleistet ist und gleichzeitig der zusätzliche Schritt des umständlichen Öffnens der Verschlussfolie vermieden wird.

Diese Aufgabe wird bei einer Vorrichtung der oben genannten Art im Wesentlichen dadurch gelöst, dass das Ventil als oleophobes Filtergewebe ausgebildet ist, welches den in dem Anschlussteil vorstehenden Lufteinlass überspannt. Eine derartige Vorrichtung ist z.B. aus der EP-A-0379047bekannt. Diese weist alle Merkmale des Oberbegriffs des Anspruchs 1 auf. Zudem ist diese Vorrichtung auch auf der in Richtung des Behälters liegenden Seite mit einer Schneideinrichtung für die den Behälter im Anlieferungszustand verschließende Folie aus Aluminium oder Kunststoff versehen, welche als kreis- oder teilkreisförmiger, von einer niedrigen Seite zu einer hohen Seite ansteigender Vorsprung ausgebildet ist.

Es ist offensichtlich, dass das oleophobe Filtergewebe vollständig die Funktion eines Rückschlagventils übernehmen kann, da das Gewebe ölige Substanzen abstößt, jedoch Luft ohne Schwierigkeiten hindurchlässt. Bei den Enteralflüssigkeiten handelt es sich grundsätzlich um Lösungen, die Lipide, Fette, Emulsionen, Multivitamine und Speiseöle enthalten. Bei der künstlichen Ernährung steht das oleophobe Filtergewebe in Berührung mit der Lösung und ermöglicht es, dass Luft in den Behälter entsprechend der Entnahme der Lösung hineingelangen kann, so dass die Strömung zum Patienten aufrechterhalten bleibt. Die zusätzlich auf dem Ventil in Richtung des Behälters vorstehende Schneideinrichtung öffnet beim Verbinden des Anschlussteils mit dem Behälter selbsttätig die Verschlussfolie, so dass ein einfaches Aufschrauben oder Aufdrücken des Anschlussteils ohne zusätzliche Manipulationen das Enteralbesteck in einen betriebsbereiten Zustand versetzt.

Da es in Einzelfällen vorkommen kann, dass beim Eindrücken der Vorrichtung nach der Erfindung in die den Behälter im Anlieferungszustand schließende Folie nicht tief genug hineingedrückt wird, kann sich unter ungünstigen Umständen eine Art Klappe aus dem Folienmaterial bilden, welche nicht vollständig von dem Folienmaterial getrennt ist. Diese Klappe kann dann möglicherweise die Schneideinrichtung und somit die Luftzufuhr wie ein Ventil erneut schließen. Folglich wird daher eine besonders vorteilhafte Ausführungsform nach der Erfindung dadurch geschaffen, dass der kreis- oder teilkreisförmige Vorsprung durch eine Anzahl von bevorzugt bis zu einem Kopfabschnitt reichenden Fenstern oder Einschnitten in eine Anzahl von Segmenten unterteilt ist. Es ist offensichtlich, dass diese Fenster oder Einschnitte in der vorstehenden Schneideinrichtung auf jeden Fall dafür sorgen, dass die Luftzufuhr offengehalten wird. Hierbei ist die spezielle Form der Fenster oder Einschnitte ohne Bedeutung, solange die gewünschte Funktion des Offenhaltens der Luftzufuhr erreicht wird. Ferner bietet diese Ausführungsform den zusätzlichen Vorteil, dass eine vollständige Entlehrung des Behälters gewährleistet ist, indem auch das üblicherweise im Flaschenhals verbleibende kleine Volumen ebenfalls ausgegeben wird.

Erfindungsgemäß ist nun das Filtergewebe in einem über den Lufteinlass stülpbaren, hülsenförmigen Gehäuse angeordnet. Dies bietet den Vorteil, dass die bisher verwendeten Anschlussteile nicht geändert werden müssen, da diese bereits einen rohrförmigen Anschlussstutzen aufweisen, auf welchem bisher das übliche Rückschlagventil befestigt war. Weiterhin ist die Schneideinrichtung auf dem Gehäuse und auf der in Richtung des Behälters liegenden Seite des Ventils angeordnet.

Im Einzelnen ist es von Vorteil, dass das Gehäuse aus Kunststoff besteht und das Filtergewebe durch Umspritzen mit diesem verbunden ist. Hierdurch ist eine kostengünstige Massenproduktion möglich.

Bei einer vorteilhaften Weiterbildung nach der Erfindung ist das Filtergewebe in dem verbreiterten Kopfabschnitt des Gehäuses angeordnet.

Im Einzelnen ist es von Vorteil, dass die Schneidvorrichtung durch Umspritzen einstückig mit dem Kopfabschnitt ausgebildet ist.

Hierbei ist es besonders bevorzugt, dass die Steigung des kreisförmigen Vorsprungs von der niedrigen Seite zur hohen Seite etwa 20° beträgt.

Bei einer vorteilhaften Weiterbildung dieser Ausführungsform ist der kreisförmige Vorsprung durch die bis zum Kopfabschnitt reichenden Einschnitte oder Fenster in sechs Segmente unterteilt.

Im Einzelnen ist es hierbei bevorzugt, dass auf dem kreisförmigen Vorsprung bzw. den Segmenten an deren Oberkante eine Schneidkante vorgesehen ist.

Bevorzugt sind ferner die aufeinander zuweisenden Kanten der Einschnitte ebenfalls mit Schneidkanten versehen. Durch diese Ausführungsform wird ein besonders müheloses Auftrennen der den Behälter verschließenden Folie gewährleistet.

Bevorzugt ist es ferner, dass die Einschnitte sich in Richtung des Kopfabschnittes verjüngen, wobei eine Verjüngung von 2° besonders bevorzugt ist. Dies erleichtert zusätzlich das Entformen der Vorrichtung nach dem Spritzgießen.

Eine weitere vorteilhafte Ausführungsform nach der Erfindung kann dadurch geschaffen werden, dass die Wandungsstärke des Vorsprungs von seinem niedrigen Ende zu seinem hohen Ende von unten nach oben abnehmend ausgebildet ist, derart, dass am hohen Ende eine Schneidkante gebildet wird.

Ferner ist es bevorzugt, dass die Innenseite des Gehäuses geringfügig konisch ausgebildet ist, um eine Klemmpassung mit dem Lufteinlass herstellen zu können.

Alternativ hierzu ist es möglich, auf der Innenseite des Gehäuses ringförmige einstückige Vorsprünge vorzusehen, um eine Schnapp- oder Reibungsverbindung mit dem Lufteinlass herzustellen.

Im Folgenden wird die Erfindung an Hand von in den Zeichnungen beispielhaft veranschaulichten Ausführungsformen näher erläutert. Es zeigt:
**Figur 1** eine schematische Ansicht eines vollständigen Enteralbesteckes, welches die Vorrichtung nach vorliegender Erfindung enthält;
**Figur 2** einen stark vergrößerten Ausschnitt von Figur 1 im Bereich des Lufteinlasses mit einer ersten Ausführungsform;
**FIGUR 3** eine perspektivische Ansicht der ersten Ausführungsform der Vorrichtung nach der Erfindung;
**Figur 4** eine Seitenansicht der Vorrichtung gemäß Figur 3 im vergrößerten Maßstab;
**Figur 5** eine Draufsicht auf die Vorrichtung gemäß Figur 4 und
**Figur 6** eine Schnittansicht der Vorrichtung gemäß den Figuren 4 und 5.
**Figur 7** einen stark vergrößerten Ausschnitt von Figur 1 im Bereich des Lufteinlasses mit einer bevorzugten zweiten Ausführungsform der Vorrichtung und der Erfindung;
**Figur 8** eine perspektivische Ansicht der Vorrichtung gemäß Figur 7 schräg von oben;
**Figur 9** eine Seitenansicht der Vorrichtung gemäß Figur 8 von der niedrigen Seite des Vorsprungs her gesehen;
**Figur 10** eine Seitenansicht der Vorrichtung gemäß Figur 9;
**Figur 11** eine Schnittansicht längs der Linie VI-VI von Figur 9;
**Figur 12** eine Schnittansicht längs der Linie VII-VII von Figur 10 und
**Figuren 13 bis 16** Schnittansichten von alternativen Ausführungsformen zur Veranschaulichung der Technik des Umspritzens.

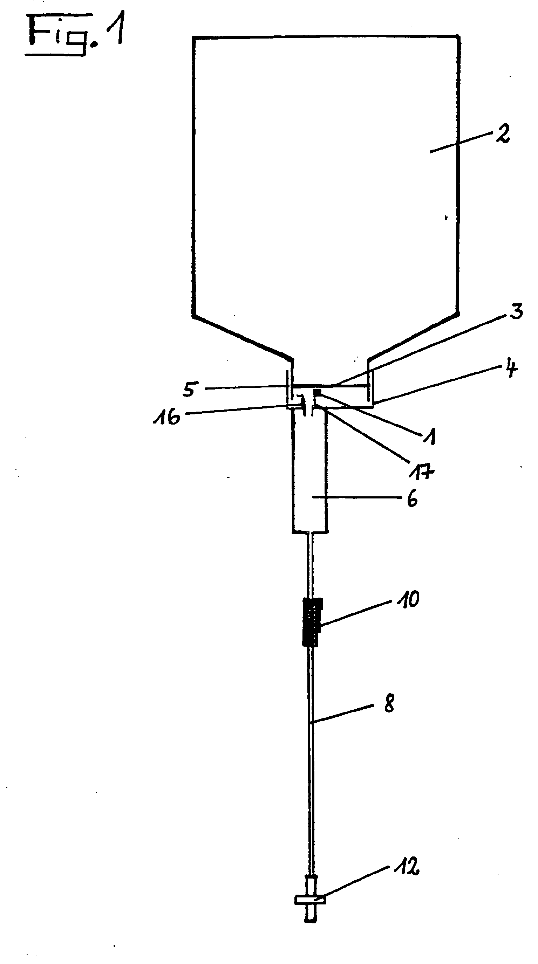

In der schematischen Ansicht gemäß Figur 1 ist die Vorrichtung 1 nach der Erfindung in einem Enteralbesteck enthalten. Das Enteralbesteck ist an einen, die Enteralflüssigkeit enthaltenden Behälter 2, welcher in dem dargestellten Zustand durch einen Folienverschluss 3 zusätzlich verschlossen ist, über einen Anschlussteil 4 angeschlossen. Hierbei handelt es sich beim Ausführungsbeispiel um eine Schraubkappe 5, welche noch nicht vollständig auf den Behälter 2 aufgeschraubt ist, so dass der Folienverschluss noch unbeschädigt ist. Die Schraubkappe 5 enthält ferner eine Tropfkammer 6, welche über eine durch eine Rollenklemme 10 gesteuerte Verbindungsleitung 8 an eine in den Patienten einzuführende Sonde 12 angeschlossen ist. Die in dem Behälter 3 enthaltene Enteralflüssigkeit wird durch Schwerkraftförderung über die Tropfkammer 6 und die Rollenklemme 10 gesteuert in den Patienten eingeleitet.

Um die Schwerkraftförderung der Enteralflüssigkeit aus dem Behälter 2 einzuleiten und aufrechtzuerhalten, enthält der Anschlussteil 4 ein Ventil 14, welches in einem Lufteinlass 16 angeordnet ist und die Luftzufuhr in den Behälter 2 entsprechend der Abgabe der Enteralflüssigkeit regelt.

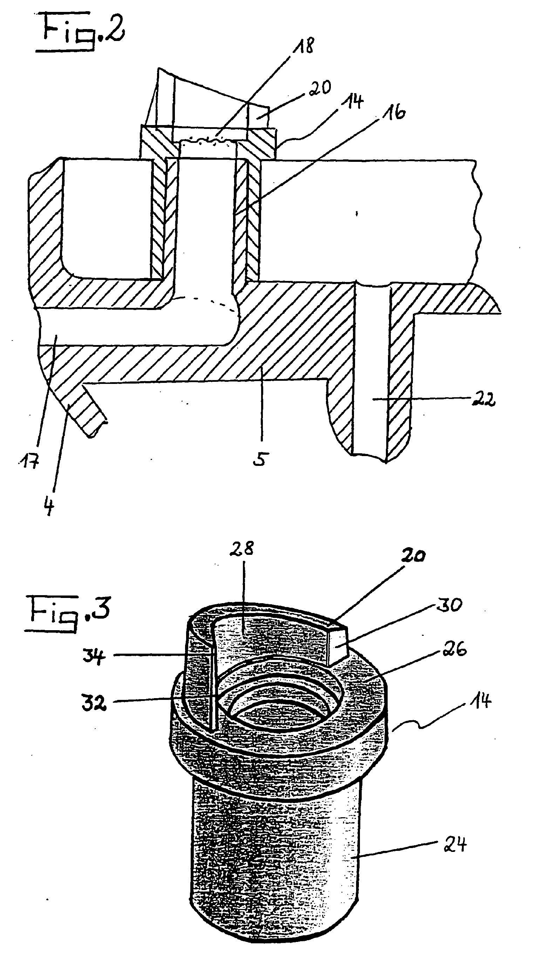

Aus den Figuren 2 bis 6 sind die Einzelheiten der Vorrichtung 1 gemäß der Erfindung näher veranschaulicht. Wie gezeigt, ist das Ventil 14 erfindungsgemäß nicht mehr als Rückschlagventil, sondern als oleophobes Filtergewebe 18 ausgebildet. Das oleophobe Filtergewebe 18 überspannt den in den Anschlussteil 4 vorstehenden Lufteinlass, so dass Luft ohne Behinderung in den Behälter 2 einströmen kann, während die ölige Enteralflüssigkeit von dem oleophoben Filtergewebe 18 abgestoßen wird.

Erfindungsgemäß ist zusätzlich das Ventil 14 auf der in Richtung des Behälters 2 liegenden Seite mit einer allgemein mit 20 bezeichneten Schneideinrichtung versehen, welche beim Verbinden des Anschlussteils 4 mit dem Behälter 2 die den Behälter 2 im Anlieferungszustand verschließende Folie aufschneidet.

Wie insbesondere aus Figur 2 ersichtlich, wobei weitere Einzelheiten in den Figuren 3 bis 6 veranschaulicht sind, ist das Filtergewebe 18 in einem über den Lufteinlass 16 stülpbaren, hülsenförmigen Gehäuse 24 angeordnet. Das Gehäuse 24 besteht bevorzugt aus Kunststoff und ist durch Umspritzen des Filtergewebes 18 mit diesem verbunden.

Das Gehäuse 24 ist mit einem verbreiterten Kopfabschnitt 26 versehen, in welchem das Filtergewebe 18. angeordnet ist.

Wie gezeigt, ist die Schneidvorrichtung 20 einstückig mit dem Kopfabschnitt 26 ausgebildet und besteht ebenfalls aus Kunststoff.

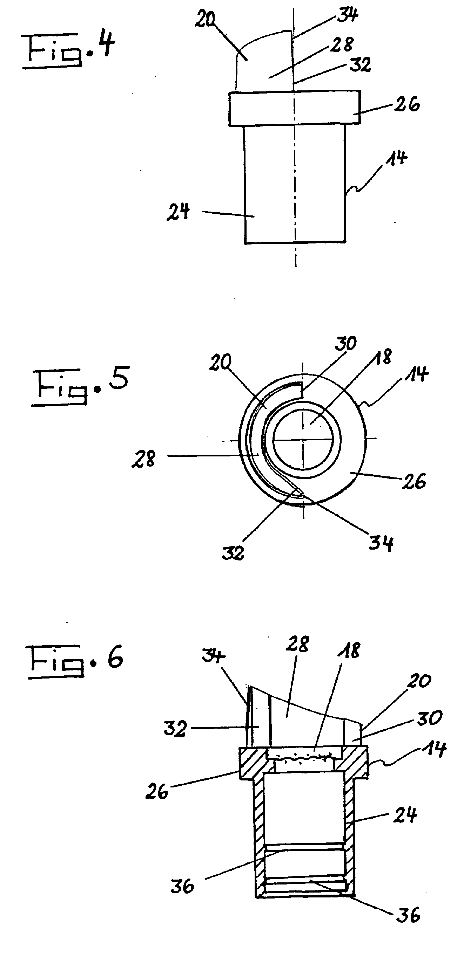

Bei der in den Figuren 3 bis 6 im Einzelnen veranschaulichten Ausführungsform der Vorrichtung 1 nach der Erfindung ist die Schneidvorrichtung als ein halbkreisförmiger Vorsprung 28 ausgebildet, welcher etwa 180° des Umfangs des Kopfabschnittes 26 einnimmt und von einem niedrigen Ende 30 zu einem hohen Ende 32 ansteigt. In der Draufsicht ist, wie in Figur 5 gezeigt, der Vorsprung 28 halbkreisförmig gestaltet.

Der Vorsprung 28 steigt in einem Winkel von etwa 160°, beim Ausführungsbeispiel 160,4° an und die Wandungsstärke des Vorsprungs 28 nimmt einerseits von seinem niedrigen Ende 30 zu seinem hohen Ende 32 ab und verjüngt sich gleichzeitig von unten nach oben. Auf diese Weise wird am hohen Ende 32 eine Schneidkante 34 ausgebildet.

Bei dem bevorzugten Ausführungsbeispiel ist entsprechend den Abmessungen des Lufteinlasses 16 in handelsüblichen Anschlussteilen 4 bzw. Schraubkappen 5 das Gehäuse 24 mit einem Außendurchmesser von etwa 8 mm versehen. Der Kopfabschnitt 26 weist einen Außendurchmesser von etwa 10,2 mm, der Vorsprung 28 einen Außendurchmesser von etwa 9,2 mm und einen Innendurchmesser von etwa 6,4 mm auf. Die Höhe des Gehäuses 24 beträgt ca. 12 mm, insbesondere 11,68 mm, und der Vorsprung 28 weist eine maximale Höhe von etwa 5 mm, insbesondere 4,87 mm, auf.

Wie insbesondere aus Figur 6 ersichtlich, ist das Gehäuse 24 im Bereich seines Unterendes auf der Innenseite mit ringförmigen, einstückigen Vorsprüngen 36 versehen, mittels derer eine Schnapp- oder Reibverbindung mit dem Lufteinlass 16 hergestellt werden kann.

Da der Lufteinlass 16, abweichend von dem zentral, im Anschlussteil 4 bzw. der Schraubkappe 5 angeordneten Flüssigkeitseinlass 22, exzentrisch ausgebildet ist und mittels eines seitlichen Kanals 17 mit der Außenluft verbunden ist, trennt die Schneidvorrichtung 20 beim Aufschrauben oder Aufdrücken des Anschlussteils 4 den Folienverschluss 3 ausreichend auf, so dass die Schwerkraftförderung der in dem Behälter 2 enthaltenen Enteralflüssigkeit beginnen kann.

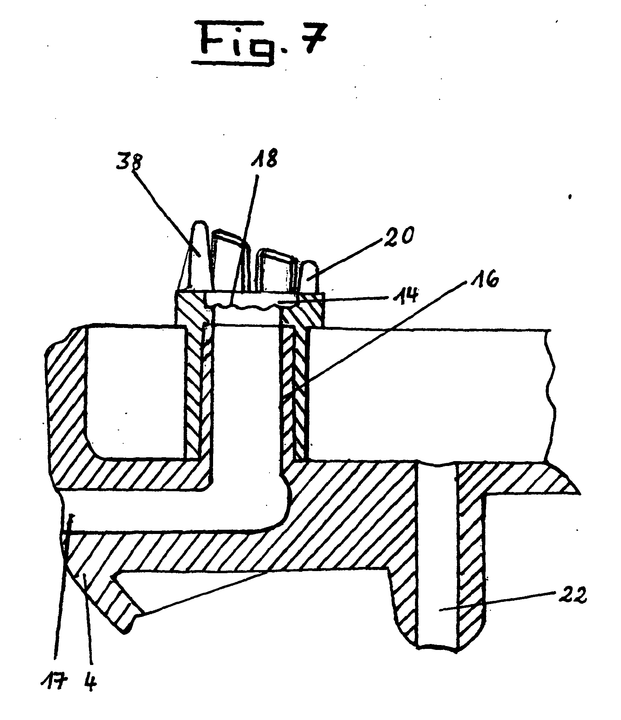

Aus den Figuren 7 bis 11 sind Einzelheiten der Vorrichtung 1 gemäß der Erfindung in einer zweiten, bevorzugten Ausführungsform näher veranschaulicht. Wie gezeigt, ist hierbei auch das Ventil 14 erfindungsgemäß nicht mehr als Rückschlagventil, sondern als oleophobes Filtergewebe 18 ausgebildet. Das oleophobe Filtergewebe 18 überspannt den in den Anschlussteil vorstehenden Lufteinlass 16, so dass Luft ohne Behinderung in den Behälter 2 einströmen kann, während die ölige Enteralflüssigkeit von dem oleophoben Filtergewebe 18 abgestoßen wird.

Gemäß dem zweiten Gesichtspunkt der Erfindung ist das Ventil 14 ebenfalls auf der in Richtung des Behälters 2 liegenden Seite mit einer allgemein mit 20 bezeichneten Schneideinrichtung versehen, welche beim Verbinden des Anschlussteils 4 mit dem Behälter 2 die den Behälter 2 im Anlieferungszustand verschließende Folie aufschneidet.

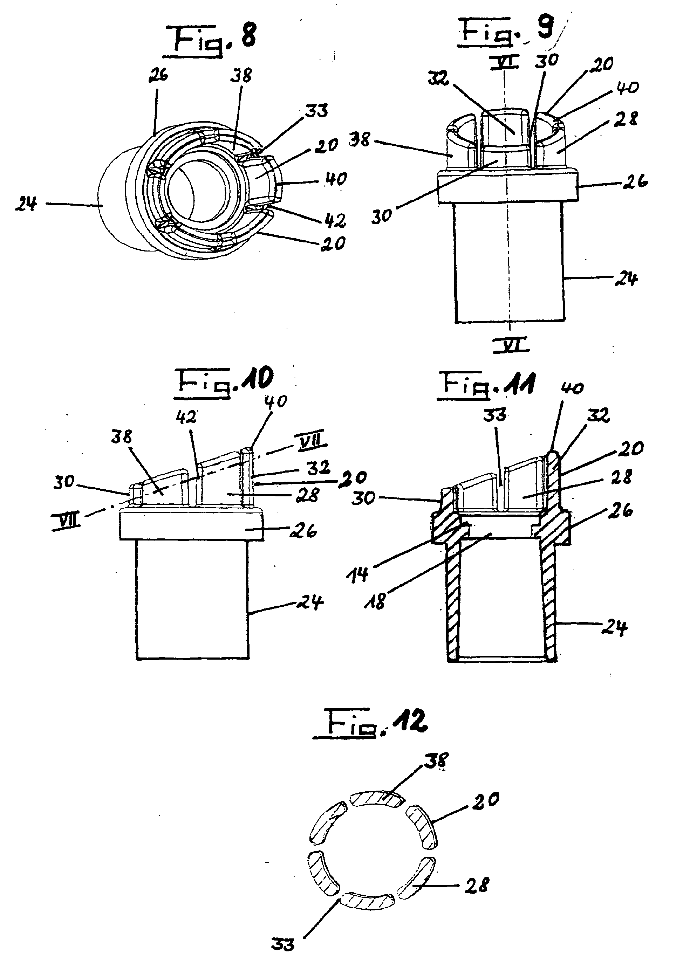

Wie insbesondere aus den Figuren 7 und 11 ersichtlich, ist das Filtergewebe 18 in einem über den Lufteinlass 16 stülpbaren, hülsenförmigen Gehäuse 24 angeordnet, wobei weitere Einzelheiten in den Figuren 8 bis 10 und 12 veranschaulicht sind. Das Gehäuse 24 besteht bevorzugt aus Kunststoff und ist durch Umspritzen des Filtergewebes 18 mit diesem verbunden.

Das Gehäuse 24 ist mit einem verbreiterten Kopfabschnitt 26 versehen, in welchem das Filtergewebe 18 angeordnet ist.

Wie gezeigt, ist die allgemein mit 20 bezeichnete Schneidvorrichtung ebenfalls durch Umspritzen einstückig mit dem Kopfabschnitt 26 ausgebildet und besteht ebenfalls aus Kunststoff.

Bei der in den Figuren 8 bis 12 im Einzelnen veranschaulichten Ausführungsform der Vorrichtung 1 nach der Erfindung ist die Schneidvorrichtung 20 als kreisförmiger Vorsprung 28 ausgebildet, welcher in einem Winkel von etwa 20° von einer niedrigen Seite 30 zu einer hohen Seite 32 ansteigt. Der kreisförmige Vorsprung 28 ist auf dem größtmöglichen Radius des Kopfabschnittes 26 angeordnet.

Der kreisförmige Vorsprung 28 ist durch eine Anzahl von bis zum Kopfabschnitt 26 heruntergeführte Einschnitte 33 in kreissegmentförmige Wandungsabschnitte oder Segmente 38 unterteilt.

Die Einschnitte 33 können alternativ auch als Fenster, Bohrungen oder dergleichen ausgebildet sein und dienen dazu, zu verhindern, dass der Lufteinlass möglicherweise wieder geschlossen wird, falls beim Eindrücken in die dem Behälter im Anlieferungszustand verschließende Folie diese nicht vollständig abgetrennt wird, sondern eine Klappe bildet, die den Einlass möglicherweise wieder verschließen könnte. Selbst wenn sich eine derartige Klappe über die Schneideinrichtung legen sollte, ist eine ungestörte Funktion durch die Einschnitte oder Fenster dennoch gewährleistet. Darüber hinaus wird hierdurch die vollständige Entlehrung des kleinen sonst im Flaschenhals des Behälters 2 zurückbleibenden Volumens gewährleistet.

Sowohl auf den freien Oberkanten der Segmente 38 als auch auf den Kanten, die sich bei den Einschnitten 33 gegenüberliegen, sind Schneidkanten 40 und 42 ausgebildet, um das Durchtrennen der Verschlussfolie zu erleichtern.

Ferner sind die Einschnitte 33 in Richtung des Kopfabschnittes 26 sich leicht verjüngend, beispielsweise in einem Winkel von 2° ausgebildet, um einerseits das Schneidverhalten zu verbessern und um andererseits das Entformen der Vorrichtung 1 nach dem Spritzgießen zu erleichtern.

Bei dem bevorzugten Ausführungsbeispiel ist entsprechend den Abmessungen des Lufteinlasses 16 in handelsüblichen Anschlussteilen 4 bzw. Schraubkappen 5 das Gehäuse 24 mit einem Außendurchmesser von etwa 8 mm versehen. Der Kopfabschnitt 26 weist einen Außendurchmesser von etwa 10 mm, der kreisförmige Vorsprung 28 einen Außendurchmesser von etwa 9 mm und einen Innendurchmesser von etwa 7 mm auf. Die Höhe des Gehäuses 24 beträgt etwa 16 mm, insbesondere 16,55 mm, auf der hohen Seite 32 und etwa 13 mm auf der niedrigen Seite 30. Entsprechend weist der Vorsprung 28 eine maximale Höhe von etwa 5 mm und eine minimale Höhe von 1,8 mm auf.

Wie insbesondere aus Figur 11 ersichtlich, ist das Gehäuse 24 im Bereich seines Unterendes auf der Innenseite mit einer geringfügigen Konizität ausgebildet, um dadurch eine Reibpassung mit dem Lufteinlass 16 herzustellen.

Da, wie aus Figur 7 ersichtlich, der Lufteinlass abweichend von dem zentral in dem Anschlussteil 4 bzw. der Schraubkappe 5 angeordneten Flüssigkeitsauslass 22 exzentrisch angeordnet ist und mittels eines seitlichen Kanals 17 mit der Außenluft verbunden ist, trennt die Schneidvorrichtung 20 beim Aufschrauben oder Aufdrücken des Anschlussteils 4 den Folienverschluss 3 ausreichend auf, so dass die Schwerkraftförderung der in dem Behälter 2 enthaltenen Enteralflüssigkeit beginnen kann.

Der guten Vollständigkeit halber wird noch darauf hingewiesen, dass zur klareren Darstellung in den Figuren 3 bis 5 und 7 das Filtergewebe 18 nicht dargestellt wurde.

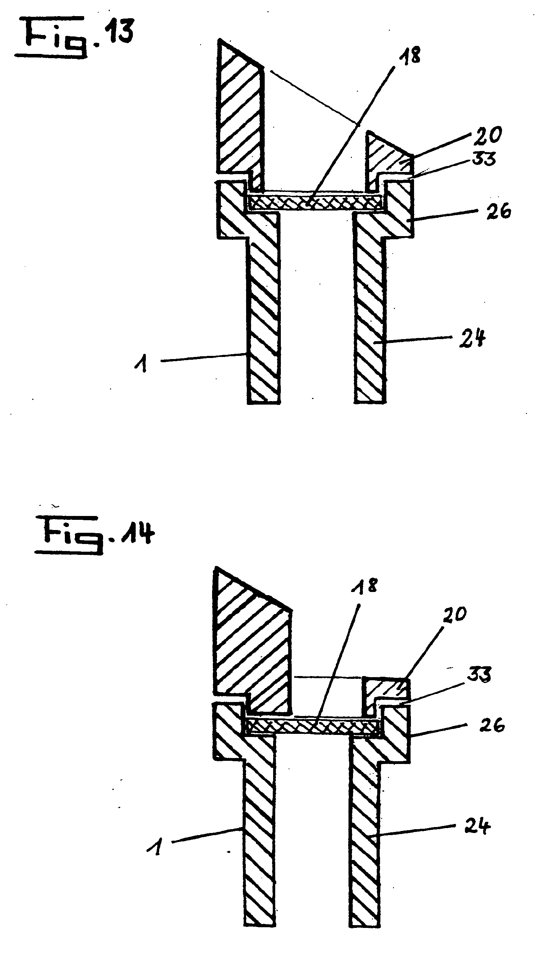

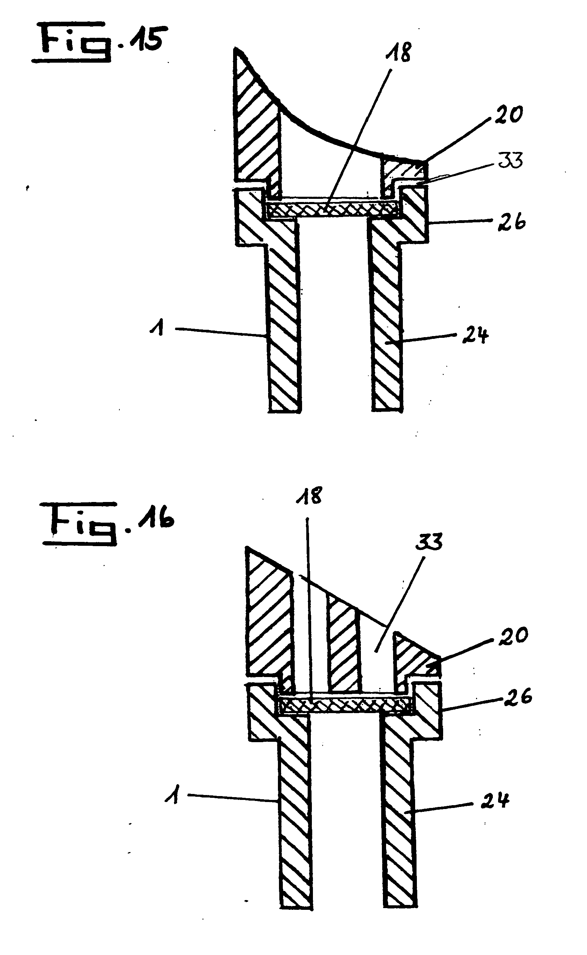

In den Figuren 13 bis 16 sind noch schematisch in seitlicher Schnittdarstellung weitere Ausführungsformen der Vorrichtung 1 dargestellt, welche die Verbindung des Gehäuses 24 mit dem Filtergewebe 18 und der Schneideinrichtung 20 durch Umspritzen erläutern, wobei die Darstellung in den Figuren für sich spricht und keiner weiteren näheren Beschreibung bedarf.

Sämtliche aus der Beschreibung, den Ansprüchen und Zeichnungen hervorgehenden Merkmale und Vorteile der Erfindung, einschließlich konstruktiver Einzelheiten und räumlicher Anordungen, können sowohl für sich als auch in beliebiger Kombination erfindungswesentlich sein.

### BEZUGSZEICHENLISTE

- 1 =: Vorrichtung
- 2 =: Behälter
- 3 =: Folienverschluss
- 4 =: Anschlussteil
- 5 =: Schraubkappe
- 6 =: Tropfkammer
- 8 =: Verbindungsleitung
- 10 =: Rollenklemme
- 12 =: Sonde
- 14 =: Ventil
- 16 =: Lufteinlass
- 17 =: Kanal
- 18 =: Filtergewebe
- 20 =: Schneideinrichtung
- 22 =: Flüssigkeitseinlass
- 24 =: Gehäuse
- 26 =: Kopfabschnitt
- 28 =: Vorsprung kreisförmig
- 30 =: niedrige Seite v. 28
- 32 =: hohe Seite v. 28
- 33 =: Einschnitte
- 34 =: Schneidkante
- 38 =: Segment
- 40 =: Schneidkante
- 42 =: Schneidkante

## Patentansprüche

1. Vorrichtung zum Einleiten von Luft in bei der künstlichen Ernährung verwendete Behälter (2), mit einem Ventil (14), welches in einem, das Enteralbesteck mit dem die Enteralflüssigkeit enthaltenden Behälter (2) verbindenden Anschlussteil (4) angeordnet ist und die Luftzufuhr entsprechend dem Verbrauch der Enteralflüssigkeit regelt, wobei das Ventil (14) als oleophobes Filtergewebe (18) ausgebildet ist, welches den in dem Anschlussteil (4) vorgesehenen Lufteinlass (16) überspannt, wobei auf der in Richtung des Behälters (2) liegenden Seite eine Schneideinrichtung (20) für den den Behälter (2) im Anlieferungszustand verschließenden Folienverschluss (3) aus Aluminium oder Kunststoff vorgesehen ist, und wobei die Schneideinrichtung (20) als kreis- oder teilkreisförmiger, von einer niedrigen Seite (30) zu einer hohen Seite (32) ansteigender Vorsprung (28) ausgebildet ist, und dass der kreisförmige Vorsprung (28) ausgebildet ist, **dadurch gekennzeichnet, dass** das Filtergewebe (18) in einem über den Lufteinlass (16) stülpbaren, hülsenförmigen Gehäuse (24) angeordnet ist, und dass die Schneideinrichtung (20) auf dem Gehäuse (24) und auf der in Richtung des Behälters (2) liegenden Seite des Ventils (14) angeordnet ist.

2. Vorrichtung nach Anspruch 1, **dadurch gekennzeichnet, dass** der kreis- oder teilkreisförmige Vorsprung (28) durch eine Anzahl von bevorzugt bis zu einem Kopfabschnitt (26) reichenden Fenstern oder Einschnitten (33) in einer Anzahl von Segmenten (36) unterteilt ist.

3. Vorrichtung nach Anspruch 1 oder 2, **dadurch gekennzeichnet, dass** das Gehäuse (24) aus Kunststoff besteht und durch Umspritzen des Filtergewebes (18) mit diesem verbunden ist.

4. Vorrichtung nach einem der vorstehenden Ansprüche, **dadurch gekennzeichnet, dass** das Filtergewebe (18) in dem verbreiterten Kopfabschnitt (26) des Gehäuses (24) angeordnet ist.

5. Vorrichtung nach einem der vorstehenden Ansprüche, **dadurch gekennzeichnet, dass** die Schneideinrichtung (20) durch Umspritzen einstückig mit dem Kopfabschnitt (26) ausgebildet ist.

6. Vorrichtung nach einem der vorstehenden Ansprüche, **dadurch gekennzeichnet, dass** die Steigung des kreisförmigen Vorsprungs (28) von der niedrigen Seite (30) zur hohen Seite (32) 20° beträgt.

7. Vorrichtung nach einem der vorstehenden Ansprüche, **dadurch gekennzeichnet, dass** der Vorsprung (28) durch die Einschnitte (33) oder Fenster in sechs Segmente (36) unterteilt ist.

8. Vorrichtung nach einem der vorstehenden Ansprüche, **dadurch gekennzeichnet, dass** der kreis- oder teilkreisförmige Vorsprung (28) bzw. die Segmente (36) mit einer oberen Schneidkante (40) versehen sind.

9. Vorrichtung nach einem der vorstehenden Ansprüche, **dadurch gekennzeichnet, dass** die Kanten der Einschnitte (33) mit einer Schneidkante (42) versehen sind.

10. Vorrichtung nach einem der vorstehenden Ansprüche, **dadurch gekennzeichnet, dass** die Einschnitte (33) sich in Richtung des Kopfabschnittes (26) verjüngen.

11. Vorrichtung nach Anspruch 10, **dadurch gekennzeichnet, dass** sich die Einschnitte (33) in Richtung, des Kopfabschnittes (26) in einem Winkel von etwa 2° verjüngen.

12. Vorrichtung nach einem der vorstehenden Ansprüche, **dadurch gekennzeichnet, dass** die Wandungsstärke des Vorsprungs (28) von seinem niedrigen Ende (30) zu seinem hohen Ende (32) und von unten nach oben abnehmend ausgebildet ist, derart, dass am hohen Ende (32) eine Schneidkante (34) gebildet ist.

13. Vorrichtung nach einem der vorstehenden Ansprüche, **dadurch gekennzeichnet, dass** die Innenseite des Gehäuses (24) eine geringe Konizität aufweist, um eine Reibpassung mit dem Lufteinlass (16) herzustellen.

14. Vorrichtung nach einem der vorstehenden Ansprüche 1 bis 13, **dadurch gekennzeichnet, dass** auf der Innenseite des Gehäuses (24) ringförmige, einstückige Vorsprünge (36) vorgesehen sind, um eine Schnapp- oder Reibungsverbindung mit dem Lufteinlass (16) herzustellen.

## Claims

1. Apparatus for introducing air into enteral containers (2), having a valve (14) positioned in a connection member (4) connecting the enteral instrument with the container (2) containing the enteral liquid and which is controlling the introduction of air corresponding to the consumption of the enteral liquid, wherein that the valve (14) is performed as an oleophobic filter fabric (18) covering the air inlet (16) provided in the connection member (4), wherein on the side lying in the direction of the container (2) a cutting device (20) for the foil closure (3) of aluminum or plastics closing the container (2) in the state of delivery is provided, wherein the cutting device (20) is performed as a circular or partly circular projection (28) rising from a low side (30) to a high side (32), **characterised in that** the filter fabric (18) is positioned in a shell-shaped housing (24) which can be mounted over the air inlet (16) and, **in that** the cutting device (20) is positioned on the housing (24 and on the side of the valve (14) facing the container (2).

2. Apparatus according to claim 1, **characterised in that** the circular or partly circular projection is separated into a number of segments (36) by a number of windows or incisions (33) preferably extending to a head section (26).

3. Apparatus according to claim 1 or 2, **characterised in that** the housing (24) is consisting of plastics and is connected with the filter fabric (18) by overmoulding the same.

4. Apparatus according to any of the preceding claims, **characterised in that** the filter fabric (18) is positioned in the broadened head section (26) of the housing (24).

5. Apparatus according to any of the preceding claims, **characterised in that** the cutting device (20) is unitary with the head section (24) by overmoulding.

6. Apparatus according to any of the preceding claims, **characterised in that** the rise of the circular projection (24) from the low side (30) to the high side (32) is 20°.

7. Apparatus according to any of the preceding claims, **characterised in that** the projection (28) is divided into six segments (36) by the incisions (33) or windows.

8. Apparatus according to any of the preceding claims, **characterised in that** the ciruclar or partly circular projection (28) or the segments (36), respectively, are provided with an upper cutting edge (40).

9. Apparatus according to any of the preceding claims, **characterised in that** the edges of the incisions (33) are provided with a cutting edge (42).

10. Apparatus according to any of the preceding claims, **characterised in that** the incisions (33) are tapering in the direction of the head section (26).

11. Apparatus according to claim 10, **characterised in that** the incisions (33) are tapering in the direction of the head section (26) in an angle of about 2°.

12. Apparatus according to any of the preceding claims, **characterised in that** the wall thickness of the projection (28) from its low end (30) to its high end (32) and from the bottom to the top is decreasing such that at the high end (32) a cutting edge (34) is formed.

13. Apparatus according to any of the preceding claims, **characterised in that** the interior of the housing (24) is having a slide conicity to provide for a friction fit with the air inlet (16).

14. Apparatus according to any of the preceding claims 1 to 13, **characterised in that** on the interior of the housing (24) there are provided annular unitary projections (36) to provide for a positive or friction fit with the air inlet (16).

## Revendications

1. Dispositif destiné à introduire de l'air dans des récipients (2) utilisés pour la nutrition artificielle, comportant une valve (14), qui est disposée dans un élément de raccord (4), par lequel l'appareillage entéral est relié au récipient (2) contenant le liquide entéral, et l'admission d'air est régulée en fonction de la consommation du liquide entéral, la valve (14) étant réalisée sous la forme d'un tissu de filtrage (18) oléophobe, qui est tendu sur la tubulure d'admission d'air (16) prévue dans l'élément de raccord (4), étant prévu un dispositif de coupe (20) pour l'opercule (3) sur le côté orienté vers le récipient (2) en aluminium ou matière plastique obturant le récipient à l'état livré, et le dispositif de coupe (20) étant réalisé sous la forme d'une saillie (28) circulaire ou partiellement circulaire avec une pente ascendante depuis un côté bas (30) vers un côté haut (32), et la saillie (28) circulaire étant formée, **caractérisé en ce que** le tissu de filtrage (18) est disposé dans un boîtier (24) en forme de manchon, pouvant être embouti sur la tubulure d'admission d'air (16), et **en ce que** le dispositif de coupe (20) est agencé sur le boîtier (24) et sur le côté de la valve (14), orienté vers le récipient (2).

2. Dispositif selon la revendication 1, **caractérisé en ce que** la saillie (28) circulaire ou partiellement circulaire est divisée en une pluralité de segments (36) par une pluralité de fenêtres ou encoches (33), s'étendant de préférence jusqu'à une partie de tête (26).

3. Dispositif selon la revendication 1 ou 2, **caractérisé en ce que** le boîtier (24) est réalisé en matière plastique et est assemblé au tissu de filtrage (18) par surmoulage de celui-ci.

4. Dispositif selon l'une quelconque des revendications précédentes, **caractérisé en ce que** le tissu de filtrage (18) est disposé dans la partie de tête (26) élargie du boîtier (24).

5. Dispositif selon l'une quelconque des revendications précédentes, **caractérisé en ce que** le dispositif de coupe (20) est réalisé par surmoulage d'un seul tenant avec la partie de tête (26).

6. Dispositif selon l'une quelconque des revendications précédentes, **caractérisé en ce que** la pente de la saillie (28) circulaire depuis le côté bas (30) jusqu'au côté haut (32) est de l'ordre de 20°.

7. Dispositif selon l'une quelconque des revendications précédentes, **caractérisé en ce que** la saillie (28) est divisée en six segments (36) par les encoches (33) ou fenêtres.

8. Dispositif selon l'une quelconque des revendications précédentes, **caractérisé en ce que** la saillie (28) circulaire ou partiellement circulaire ou les segments (36) sont munis d'une arête de coupe (40) supérieure.

9. Dispositif selon l'une quelconque des revendications précédentes, **caractérisé en ce que** les bords des encoches (33) sont munis d'une arête de coupe (42).

10. Dispositif selon l'une quelconque des revendications précédentes, **caractérisé en ce que** les encoches (33) se rétrécissent en direction de la partie de tête (26).

11. Dispositif selon la revendication 10, **caractérisé en ce que** les encoches (33) se rétrécissent en direction de la partie de tête (26) selon un angle de 2° environ.

12. Dispositif selon l'une quelconque des revendications précédentes, **caractérisé en ce que** l'épaisseur de paroi de la saillie (28) depuis son extrémité basse (30) vers son extrémité haute (32) est réalisée en diminuant du bas vers le haut, de telle sorte qu'une arête de coupe (34) est réalisée sur l'extrémité haute (32).

13. Dispositif selon l'une quelconque des revendications précédentes, **caractérisé en ce que** la face intérieure du boîtier (24) présente une faible conicité pour réaliser un ajustement par frottement avec la tubulure d'admission d'air (16).

14. Dispositif selon l'une quelconque des revendications 1 à 13, **caractérisé en ce que** sur la face intérieure du boîtier (24) sont prévues des saillies (36) annulaires d'un seul tenant pour réaliser un assemblage par encliquetage ou frottement avec la tubulure d'admission d'air (16).
